# EUROPÄISCHE PATENTSCHRIFT

(11) **EP 1 037 613 B1**
(45) Veröffentlichungstag und Bekanntmachung des Hinweises auf die Patenterteilung: **23.01.2002**
(21) Anmeldenummer: 98966581.5
(22) Anmeldetag: 17.12.1998
(51) Int. Cl.: A61K 9/16, A61K 9/20, A61K 35/78

(54) **VERFAHREN ZUM HERSTELLEN VON EXTRAKT ENTHALTENDEN PHARMAZEUTISCHEN FORMULIERUNGEN**
METHOD FOR PRODUCING PHARMACEUTICAL FORMULATIONS CONTAINING AN EXTRACT
PROCEDE POUR PRODUIRE DES FORMULATIONS PHARMACEUTIQUES CONTENANT UN EXTRAIT

(30) Priorität: 18.12.1997 DE 19758100
(43) Veröffentlichungstag der Anmeldung: 27.09.2000
(73) Patentinhaber: Lichtwer Pharma AG, 13435 Berlin (DE)
(72) Erfinder: SCHMIDT, Peter, C., D-72074 Tübingen (DE); ROCKSLOH, Karin, D-72138 Kirchentellinsfurt (DE); MÜLLER, Wolfgang, D-16548 Glienicke (DE); REHER, Markus, D-16548 Glienicke (DE); ABU ABED, Salah, D-13469 Berlin (DE)
(74) Vertreter: Bröseke, Eribert, Dipl.-Ing.
(86) Internationale Anmeldenummer: DE9803784
(87) Internationale Veröffentlichungsnummer: WO9932090

(56) Entgegenhaltungen:
- EP-A- 0 348 509
- EP-A- 0 530 833
- DE-A- 4 139 118

## Beschreibung

Die Erfindung betrifft ein Verfahren zur Herstellung von Extrakten enthaltenden pharmazeutischen Formulierungen, wie Tabletten, Filmtabletten, Dragées u.a.

Pflanzliche Arzneimittel enthalten in festen Zubereitungen Trockenextrakte aus Drogen, die im allgemeinen durch Extraktion mit Alkohol/Wasser-Gemischen und anschließendes Eindampfen und Trocknen hergestellt werden. Die Dosierung dieser Extrakte kann pro Einzeldosis in weiten Bereichen schwanken. Die Extrakte sind hygroskopisch und lassen sich schlecht zu pharmazeutischen Formulierungen wie Tabletten bzw. Kernen für überzogene Tabletten verpressen. Gewöhnlich enthalten die Extrakte Schleimstoffe, die den späteren Zerfall der Tabletten, Filmtabletten bzw. Dragees behindern.

Nach dem Stand der Technik werden pharmazeutische Formulierungen, wie Tabletten bzw. Tablettenkerne für Filmtabletten und Zuckerdragées auf dem Wege der Direkttablettierung bzw vorheriger Granulierung der Wirk- und gegebenenfalls Hilfsstoffe hergestellt. Die Direkttablettierung ist im Verfahrensablauf einfach, da die Bestandteile lediglich gemischt und zu Tabletten verpreßt werden. Dadurch tritt keine Feuchtigkeitsbelastung während der Granulation auf und Hydrolysevorgänge werden vermieden. Ebenso wird eine Temperaturbelastung während der Trocknung der Granulate ausgeschlossen.

Bei vielen Extrakten versagt die Direkttablettierung jedoch häufig wegen der schlechten Fließfähigkeit und der schlechten Verpreßbarkeit der Extrakte. Bei der Granulation der Wirkstoffe werden diese mit Lösungsmitteln oder Bindemittellösungen gemischt und auf bekannten Vorrichtungen granuliert und anschließend getrocknet.

Die Granulation durch Befeuchten mit Lösungsmitteln bzw. dem Einsatz von Bindemittellösungen bringt eine Erhöhung der Teilchengröße und Vereinheitlichung der Teilchengrößenverteilung der Arzneistoffe mit sich. Gleichzeitig wird die Fließfähigkeit des Granulates gegenüber den Ausgangssubstanzen verbessert. Ebenso läßt sich eine größere Dosiergenauigkeit auf der Tablettenmaschine erreichen.

Bei extrakthaltigen Präparaten kann die wässrige Granulation nur selten angewendet werden, da Extrakte aufgrund ihrer hohen Löslichkeit und hohen Hydrophilie sehr schnell zum Verkleben bzw. zum Verschmieren während des Granulationsvorgangs neigen.

Ein Ausweichen auf organische Lösungsmittel zur Granulation ist aus toxikologischen und umwelttechnischen Gründen heutzutage nur schwer realisierbar.

Aus der EP A 0 530 833 ist ein Verfahren zur Herstellung von Kampo-Extrakt-enthaltener Hartgelantine-Kapseln bekannt. Dabei wird das Kampo-Extrakt in einer ersten Stufe zusammen mit Mengen zwischen 0,5 bis 1 GEW % Magnesiumstearat und anderen üblichen Hilfstoffen komprimiert, gebrochen und klassiert. Das erhaltene Granulat wird mit einer weiteren Menge Magnesiumstearat vermischt und in Hartgelatine-Kapseln abgefüllt. Ein Zusatz von Magnesiumstearat zu den bereits vorgefertigten granulierten Extrakt, also in die äußere Phase, ist obligatorisch. In dieser Schrift wird darauf abgestellt, daß die Zugabe des Magnesiumstearat nicht als Schmiermittel erfogt, sonder zur Verbesserung der Auflöseeigenschaften der pharmazeutischen Hartgelatinekapseln.

Der Erfindung liegt die Aufgabe zugrunde, Extrakte so aufzubereiten, daß sie sich bei einer Extraktmenge von 100 bis 1000 mg pro Einzeldosis gut in pharmazeutische Formulierungen, wie Tabletten, Filmtabletten, Dragées u.ä. einarbeiten lassen und in angemessener Zeit aus den pharmazeutischen Formulierungen freigegeben werden.

Erfindungsgemäß wird die Aufgabe durch die kennzeichnenden Merkmale des Anspruches 1 gelöst. Nach dem erfindungsgemäßen Verfahren werden die Extraktpulver zusammen mit einem Schmiermittel kompaktiert. Dazu wird das Extraktpulver mit Mengen von 0,5 bis 10 Gew. % an Schmiermitteln vermischt und kompaktiert. Vorzugsweise wird mit Mengen von 2-5 Gew.% an Schmiermitteln bei der Kompaktierung gearbeitet. Insbesondere sind Mengen von 3 Gew.% Schmiermittel geeignet.

Die Kompaktierung erfolgt auf dafür geeigneten Vorrichtungen. So ist es möglich, die Mischung auf einem Kompaktor zwischen zwei Walzen zu einer Schülpe zu verpressen. Bei der Herstellung der Kompaktate auf dem Kompaktor wird mit einer Anpreßkraft von 20 bis 120 kN und einer Walzendrehzahl von 5 bis 15 Umdrehungen/min gearbeitet. Vorzugsweise Anpreßkräfte liegen bei 35 bis 65 kN. Insbesonders geeignete Anpreßkräfte liegen bei 42 bis 48 kN. Bei einer Arbeitsbreite der Walze von 5 cm ergibt sich eine spezifische Anpreßkraft von 4 bis 25 kN/cm.

Zur Verbesserung der Verpressung kann der Vorrichtung für die eigentliche Kompaktierung eine Vorverdichtungsvorrichtung vorgeschaltet werden. So ist es möglich, beim Einsatz eines Kompaktors einen solchen mit vorgeschalteter Vorverdichtungsschnecke einzusetzen. Die mit dem Kompaktor verbundene Vorverdichtungsschnecke arbeitet mit einer Schnekkendrehzahl von 20 bis 60 Umdrehungen/min. Vorzugsweise Werte für die Umdrehungszahl liegen bei 25 bis 35 Umdrehungen/min. Die erhaltene Schülpe wird anschließend trocken zu einem Granulat definierter Korngröße zerkleinert. Vorzugsweise erfolgt die Zerkleinerung und Granulation auf einer Siebmaschine.

Das erhaltene Granulat wird nach Zugabe von Hilfsstoffen, wie Trockenbindemitteln, Sprengmitteln und ggf. Fließregulierungsmitteln zu pharmazeutischen Formulierungen verpreßt. Durch die Kompaktierung des Extraktes mit einem Schmiermittel wird in der äußeren Phase kein weiteres Schmiermittel mehr benötigt. Das Hinzufügen eines die Kapillarkräfte erhöhenden Fließregulierungsmittels in der Äußeren Phase ergibt pharmazeutische Formulierungen, die im Vergleich zu nach herkömmlichen Verfahren hergestellten pharmazeutischen Formulierungen eine kürzere Zerfallzeit bei höherer Bruchfestigkeit aufweisen. Es werden 0,3 bis 2,5 Gew.%, vorzugsweise 1 Gew.% Fließregulierungsmittel zugesetzt.

Ebenso bringt die Zugabe eines Sprengmittels in die äußere Phase bei mit kompaktiertem Extrakt hergestellten pharmazeutischen Formulierungen wirksamere pharmazeutische Formulierungen als bei einer Direkttablettierung des Extraktes. Es werden 2 bis 10 Gew.%, vorzugsweise 4% Sprengmittel zugesetzt.

Als Schmiermittel werden bei der Kompaktierung nach dem erfindungsgemäßen Verfahren für die Extrakte Metallseifen, wie Magnesiumstearat, Zinkstearat und Calciumarachinat sowie Triglyceride, vorzugsweise Glyceroltristearat, bzw. Gemische aus Mono-, Di- und Triglyceriden sowie hydriertes Rhizinusöl, Stearinsäure, Talkum, Natriumbenzoat, Fumarsäure, Adipinsäure, Leucin und Natriumstearylfumarat eingesetzt.

Als Hilfsstoffe für die Herstellung der pharmazeutischen Formulierungen können als Füll- und Bindemittel für die Direkttablettierung mikrokristalline Cellulose, mikrofeine Cellulose, Lactosen, Calciumphosphate, teilabgebaute Stärken, Calciumsulfat und Calciumcarbonat verwendet werden.

Als Sprengmittel eignen sich Alginsäure, Bentonit, Calciumcarboxymethylcellulose, quervernetzte Carboxymethylcellulose, mikrokristalline Cellulose, Guar-Gummi, Hydroxypropylcellulose mit niedrigem Substitutionsgrad mit oder ohne Quervernetzung, Ionenaustauscher, Natriumcarboxymethylstärke, quervernetztes Polyvinylpyrrolidon, Stärke sowie teilabgebaute Stärke.

Überraschenderweise wurde gefunden, daß durch die Einarbeitung des Schmiermittels in das Kompaktat Granulate erzeugt werden, die eine verbesserte Fließfähigkeit, herabgesetzte Hygroskopizität, gute Verpreßbarkeit, einen verbesserten Zerfall, eine verbesserte Bruchfestigkeit und eine herabgesetzte Friabilität der entstehenden pharmazeutischen Formulierungen besitzen.

Die Erfindung soll anhand von Beispielen näher erläutert werden.

Bei den in den Beispielen genannten Handelsprodukten handelt es sich im einzelnen bei

| Bezeichnung der Substanz | Handelsprodukt der Firma | Zusammensetzung |
|---|---|---|
| Cellactose | Meggle Marke- | 75 % -Lactose-Monohydrat 25 % Cellulosepulver |
| Tablettierhilfsmittel K | E. Merck KGaA | Cellulosepulver |
| Ac-di-Sol | Lehmann & Voss & Co. | quervernetzte Carboxymethylcellulose |
| Dynasan 118 | Hüls AG | Glyceroltristearat |
| Avicel PH 101 | Lehmann & Voss & Co. | mikrokristalline Cellulose |
| Cab-O-Sil | Cabot GmbH | kolloidales Siliciumdioxid |
| Kollidon CL | BASF AG | quervernetztes Polyvinylpyrrolidon |

### Beispiel 1:

In diesem Beispiel sollen die erhaltenen Werte für Bruchfestigkeit und Zerfallzeiten der pharmazeutischen Formulierungen von kompaktiertem und nicht kompaktiertem Extrakt gegenübergestellt werden.

Zur Herstellung des Kompaktates aus Extraktpulver und Schmiermittel werden dem Extraktpulver 3 % Magnesiumstearat als Schmiermittel hinzugegeben und in einem geeigneten Mischer vermischt.

Aus dieser Mischung werden die Kompaktate auf einem Pharmapaktor L 200/50 P hergestellt. Der Kompaktor ist mit konkaven Glattwalzen ausgestattet, die Arbeitsbreite beträgt 5 cm, und es wird mit einer Anpreßkraft von 45 kN gearbeitet. Daraus ergibt sich eine spezifische Anpreßkraft von 9 kN/cm Walzenbreite. Die Walzendrehzahl beträgt 10,3 Umdrehungen/min. Der Kompaktor ist ferner mit einer zylindrischkonischen Vorverdichtungsschnecke zur Entlüftung und zur Vorverdichtung der Pulvermasse ausgestattet. Die Schnekkendrehzahl beträgt 25 Umdrehungen/min. Bei der Verarbeitung der Extrakte ohne Magnesiumstearat werden diese direkt auf den Kompaktor gegeben.

Die erhaltene Schülpe wird anschließend auf einer Siebmühle, z.B. Siebmühle FC 200 mit einem Sieb der Maschenweite 1,5 mm zu Granulaten zerkleinert.

Die Herstellung der pharmazeutischen Formulierung, hier Tabletten, erfolgt auf einer Rundlauftablettenpresse Korsch Pharma 103 mit 3 Stempelwerkzeugen bei 20 Umdrehungen/min mit unterschiedlicher Preßkraft.

Die zu verarbeitenden Rezepturen bestehen
bei dem nichtkompaktierten Extrakt a) aus:

| Bestandteile | |
|---|---|
| Nativer Extrakt (94 %) | 531,9 mg |
| Cellactose | 99,9 mg |
| Tablettierhilfsmittel K | 27,1 mg |
| Ac-di-Sol | 28,0 mg |
| Dynasan 118 | 14,0 mg |
| Gesamtmasse | 700,0 mg |

bei dem kompaktierten Extrakt b) aus

| Bestandteile | |
|---|---|
| Kompaktierter Extrakt (mit 3 % Magnesiumstearat als Schmiermittel) | 537,3 mg |
| Avicel PH 101 | 127,7 mg |
| Ac-di-Sol | 28,0 mg |
| Cab-O-Sil | 7,0 mg |
| Gesamtmasse | 700,0 mg |

Die erhaltenen Produkte wurden hinsichtlich ihrer Bruchfestigkeit und Zerfallzeit bei der Anwendung gegenübergestellt. Es wurden folgende Werte erhalten:

| | Nichtkompak. Extrakt | | | Komp. Extrakt | | |
|---|---|---|---|---|---|---|
| Preßkraft (kN) | 3,7 | 6,1 | 7,6 | 3,4 | 5,5 | 7 |
| Bruchfestigkeit N | 34,3 | 83,3 | 119,4 | 31,3 | 69,8 | 101,1 |
| Zerfall (min) | 37 | 44 | 47,5 | 17,5 | 31,5 | 33,5 |

Bei allen Beispielen wurde die Bruchfestigkeit nach dem EuAB 1997 (Europäisches Arzneibuch 1997) mit einem Schleuniger-6 D-Bruchfestigkeitstester bestimmt. Ebenso sind die Zerfallzeiten nach EuAB 1997 bestimmt worden.

Die Ergebnisse zeigen, daß mit nicht kompaktiertem Extrakt und Schmiermittel in der äußeren Phase in allen Fällen wesentlich längere Zerfallzeiten erhalten werden, als mit kompaktiertem Extrakt, der einen Zusatz von 3 % Magnesiumstearat im Kompaktat enthält. Insbesondere bei niederen Preßkräften sind die Unterschiede zwischen den Zerfallzeiten für den nicht kompaktierten Extrakt gegenüber dem kompaktierten Extrakt besonders deutlich.

### Beispiel 2:

In diesem Beispiel werden die im Beispiel 1 angesprochenen Werte für die Bruchfestigkeit und Zerfallzeit von kompaktiertem Extrakt mit bzw. ohne Magnesiumstearat im Kompaktat gegenübergestellt.

Die verwendeten Kompaktate wurden, wie in Beispiel 1 beschrieben, hergestellt. Die Tablettierung erfolgte ebenfalls wie im Beispiel 1 beschrieben.

Die Rezepturen bestehen aus
a) Kompaktat mit 5 % Magnesiumstearat
b) Kompaktat ohne Magnesiumstearat (2 % in äußerer Phase),
c) Kompaktat ohne Magnesiumstearat (1 % in äußerer Phase).

| | a) | b) | c) |
|---|---|---|---|
| Kompakt. Extrakt | 315,8 mg | 300,0 mg | 300,0 mg |
| Avicel PH 101 | 125,8 mg | 132,4 mg | 137,0 mg |
| Kollidon CL | 18,4 mg | 18,4 mg | 18,4 mg |
| Magnesiumstearat | - | 9,2 mg | 4,6 mg |
| Gesamtmasse pro Tablette | 460,0 mg | 460,0 mg | 460,0 mg |

Es wurden folgende Ergebnisse erzielt:

| | a) | | b) | | c) | |
|---|---|---|---|---|---|---|
| Preßkraft (kN) | 6 | 7,6 | 7,1 | 8,6 | 7,7 | 9,4 |
| Bruchfestigkeit (N) | 53,7 | 82,8 | 49,4 | 60,8 | 48,9 | 60,4 |
| Zerfallzeit (min) | 12,5 | 18,5 | 20,0 | 30,0 | 38,5 | 44 |

Die resultierenden Zerfallszeiten zeigen, daß bei Einarbeitung von 5 % Magnesiumstearat in das Kompaktat wesentlich kürzere Zerfallszeiten erhalten werden als mit 1 bzw. 2 % Magnesiumstearat in der äußeren Phase bei gleichzeitiger Abwesenheit von Magnesiumstearat im Kompaktat. Deshalb ist die Anwendung von Magnesiumstearat in der äußeren Phase nicht notwendig, sofern das Kompaktat ein Schmiermittel enthält. Als weiterer Vorteil ergibt sich trotz hoher Magnesiumstearatmenge im Kompaktat eine kürzere Zerfallzeit der Tabletten.

### Beispiel 3:

In diesem Beispiel werden Zerfallzeiten von Tabletten gegenübergestellt, die unterschiedliche Schmiermittel und unterschiedliche Mengen an Schmiermitteln im Kompaktat enthalten.

Als Schmiermittel wurden zum einen Magnesiumstearat und zum anderen Dynasan 118 eingesetzt.

Die Herstellung der Kompaktate und die Tablettierung erfolgte wie in Beispiel 1.

Die Rezepturen enthalten a) 5 %, b) 3 % und c) 1 % Schmiermittel im Kompaktat.

| | a) | b) | c) |
|---|---|---|---|
| Kompakt. Extrakt | 335,9 mg | 329,0 mg | 322,3 mg |
| Avicel PH 101 | 105,7 mg | 112,6 mg | 120,1 mg |
| Ac-di-Sol | 18,4 mg | 18,4 mg | 18,4 mg |

Es wurden folgende Ergebnisse erhalten:

| | 5 %Mg- 5 stearat | % Dynasan | 3 % Mg-stearat | 3 % Dynasan | 1 % Mg-stearat | 1 % Dynasan |
|---|---|---|---|---|---|---|
| | | im Kompaktat | | | | |
| Preßkraft (kN) | 13,1 | 12,8 | 11,9 | 16,2 | 13,2 | 13,2 |
| Bruchfestigkeit | 81,8 | 80,4 | 85,4 | 96,8 | 89,5 | 89,7 |
| Zerfall (min) | 37,0 | 31,5 | 28,0 | 24,5 | 26,5 | 25,5 |

Der Vergleich der Schmiermittel Magnesiumstearat bzw. Dynasan 118 im Kompaktat zeigt, daß bei gleichen Bruchfestigkeiten die Tabletten mit Dynasan 118 im Mittel kürzere Zerfallzeiten ergeben.

Eine zusätzliche Schmierung in der äußeren Phase ist nicht erforderlich.

Die erhaltenen Ergebnisse zeigen weiter, daß bei geringeren Schmiermittelanteilen in der Rezeptur für das Kompaktat die Unterschiede zwischen den erhaltenen Zerfallzeiten für die verschiedenen Schmiermittel sich angleichen.

Ausreichend kurze Zerfallzeiten bei sicherer Schmierung in allen Rezepturen werden bei Schmiermittelzusätzen von 3 % erreicht.

### Beispiel 4:

In diesem Beispiel wird der Einfluß von Sprengmittelzusätzen hinsichtlich der Zerfallzeit bei unterschiedlicher Bruchfestigkeit untersucht.

Die Herstellung der Kompaktate und der Tabletten erfolgte wie in Beispiel 1 beschrieben.

Die Rezepturen bestehen aus:

| | | | |
|---|---|---|---|
| Komp. Extrakt (mit 5% Mg.-stearat) | 315,8 mg | 315,8 mg | 315,8 mg |
| Avicel PH 101 | 144,2 mg | 125,8 mg | 125,8 mg |
| Ac-di-Sol | - | - | 18,4 mg |
| Kollidon CL | - | 18,4 mg | - |
| Gesamtmasse | 460,0 mg | 460,0 mg | 460,0 mg |

Es wurden folgende Ergebnisse erhalten:

| | ohne Sprengmittelzusatz | | mit 4% Kollidon CL | | mit 4% Ac-di-Sol | |
|---|---|---|---|---|---|---|
| Preßkraft (kN) | 3,8 | 8,7 | 6 | 7,6 | 6,3 | 7,6 |
| Bruchfestigkeit (N) | 36,9 | 100,2 | 53,7 | 82,8 | 56,0 | 71,6 |
| Zerfall (min) | 19,0 | 85,0 | 12,5 | 18,5 | 19,0 | 26,5 |

Die Ergebnisse zeigen, daß der Zusatz von 4 % Kollidon CL als Sprengmittel in der äußeren Phase eine signifikante Reduktion der Zerfallzeiten gegenüber einer Tablette ohne Sprengmittelzusatz bewirkt. Die Wirkung von 4 % Ac-di-Sol als Sprengmittel ist bei niederen Preßkräften nicht so ausgeprägt wie die von Kollidon CL, ergibt jedoch bei höheren Preßkräften und härteren Tabletten eine deutliche Verbesserung gegenüber einer Tablette ohne Sprengmittelzusatz.

### Beispiel 5:

Mit diesem Beispiel wird der Einfluß eines Fließregulierungsmittelzusatzes in der äußeren Phase hinsichtlich der Bruchfestigkeit und der Zerfallzeit untersucht.

Die Herstellung der Kompaktate und der Tabletten erfolgte wie in Beispiel 1 beschrieben.

Die Rezepturen bestehen aus:

| | | |
|---|---|---|
| Komp. Extrakt (mit 1 % Mg-stearatzusatz im Kompaktat) | 322,3 mg | 322,3 mg |
| Avicel PH 101 | 114,7 mg | 119,3 mg |
| Kollidon CL | 18,4 mg | 18,4 mg |
| Cab-O-Sil M 5 | 4,6 mg | - |

Es wurden folgende Ergebnisse erhalten:

| | Preßmischung mit Fließregulierungsmittel | | | Preßmischung ohne Fließregulierungsmittel | | |
|---|---|---|---|---|---|---|
| Preßkraft (kN) | 4,3 | 6,5 | 9,2 | 7,6 | 9,7 | 13,2 |
| Bruchfestigkeit(N) | 36,8 | 76,7 | 99,8 | 47,2 | 64,0 | 89,5 |
| Zerfall (min) | 9,5 | 15,5 | 25,0 | 13,5 | 21,0 | 26,5 |

Die Ergebnisse zeigen, daß durch den Zusatz von Cab-O-Sil M5 als Fließregulierungsmittel eine deutliche Erhöhung der Bruchfestigkeit ohne größere Änderung der Zerfallzeiten zu beobachten ist. Über den gesamten Preßkraftbereich hinweg resultieren bei einem Zusatz von 1 % Cab-O-Sil als Fließregulierungsmittel härtere Tabletten.

## Patentansprüche

1. Verfahren zum Herstellen von schnell zerfallenden pharmazeutischen Formulierungen mit Extrakt enthaltenden Präparaten,
**dadurch gekennzeichnet,**
**daß** in einer ersten Stufe die Extraktpulver zusammen mit einem Schmiermittel in Mengen von 0,5 - 10 Gew.% mittels einer geeigneten Vorrichtung trocken zu einer Schülpe kompaktiert, anschließend zu einem Granulat gebrochen, klassiert und die erhaltenen Kompaktate in einer zweiten Stufe mit den üblichen Hilfsstoffen, wie Füll-, Gleit-, Spreng-, Fließregulierungsmittel u.a. Zusatzmitteln zu pharmazeutischen Formulierungen verpreßt werden.

2. Verfahren nach Anspruch 1, **dadurch gekennzeichnet, daß** als Vorrichtung für die Verpressung ein Kompaktor eingesetzt wird, während für die Zerkleinerung und Granulation ein Siebgranulator benutzt wird.

3. Verfahren nach Anspruch 1 und 2, **dadurch gekennzeichnet, daß** beim Verpressen des Extraktpulvers im Kompaktor mit einem Anpreßdruck von 20 bis 120 kN, vorzugsweise 35 bis 65 kN und insbesondere 42 bis 48 kN und einer Walzendrehzahl von 5 bis 15 Umdrehungen/min gearbeitet wird.

4. Verfahren nach mindestens einem der Ansprüche 1 bis 3, **dadurch gekennzeichnet, daß** der Kompaktor zur Vorverdichtung eine Vorverdichtungsschnecke aufweist, die mit einer Schneckendrehzahl von 20 bis 60 Umdrehungen/min, vorzugsweise 25 bis 35 Umdrehungen/min betrieben wird.

5. Verfahren nach mindestens einem der Ansprüche 1 bis 4, **dadurch gekennzeichnet, daß** die Extraktmenge pro Einzeldosis 100 bis 1000 mg beträgt.

6. Verfahren nach mindestens einem der Ansprüche 1 bis 5, **dadurch gekennzeichnet, daß** die Menge an Schmiermittel im Kompaktat vorzugsweise 2 bis 5 Gew.%, insbesondere 3 Gew.% beträgt.

7. Verfahren nach mindestens einem der Ansprüche 1 bis 6, **dadurch gekennzeichnet, daß** als Schmiermittel Magnesiumstearat eingesetzt wird.

8. Verfahren nach mindestens einem der Ansprüche 1 bis 6, **dadurch gekennzeichnet, daß** als Schmiermittel ein Glycerolfettsäureester, auch Partialester, vorzugsweise Glyceroltristearat eingesetzt wird.

9. Verfahren nach mindestens einem der Ansprüche 1 bis 8, **dadurch gekennzeichnet, daß** bei der Tablettierung der Mischung 2 bis 10 Gew.%, vorzugsweise 4 Gew.% an Sprengmitteln der äußeren Phase zugesetzt werden.

10. Verfahren nach mindestens einem der Ansprüche 1 bis 9, **dadurch gekennzeichnet, daß** bei der Tablettierung der Mischung 0,3 bis 2,5 Gew.%, vorzugsweise 1 Gew.% eines Fließregulierungsmittels der äußeren Phase zugesetzt werden.

## Claims

1. Process for the production of rapidly disintegrating pharmaceutical formulations with extract-containing products,
**characterized in that**
in a first stage the extract powders are compacted dry together with a lubricant in amounts of 0.5 - 10% by weight by means of a suitable device to give flakes and subsequently crushed to granules and classified, and the resulting compacts are compressed in a second stage with conventional excipients such as bulking agents, glidants, disintegrants, flow regulators and other additives to pharmaceutical formulations.

2. Process according to Claim 1, **characterized in that** the device employed for the compression is a compactor, while a screening granulator is used for the reduction in size and granulation.

3. Process according to Claim 1 and 2, **characterized in that** a pressure of from 20 to 120 kN, preferably 35 to 65 kN and, in particular, 42 to 48 kN, with the rolls revolving at from 5 to 15 revolutions/min, is used for compression of the extract powder in the compactor.

4. Process according to at least one of Claims 1 to 3, **characterized in that** the compactor has a precompaction screw for precompaction, which is operated with the screw revolving at from 20 to 60 revolutions/min, preferably 25 to 35 revolutions/min.

5. Process according to at least one of Claims 1 to 4, **characterized in that** the amount of extract per single dose is from 100 to 1000 mg.

6. Process according to at least one of Claims 1 to 5, **characterized in that** the amount of lubricant in the compact is preferably from 2 to 5% by weight, in particular 3% by weight.

7. Process according to at least one of Claims 1 to 6, **characterized in that** magnesium stearate is employed as lubricant.

8. Process according to at least one of Claims 1 to 6, **characterized in that** a glycerol fatty acid ester, also partial ester, preferably glycerol tristearate, is employed as lubricant.

9. Process according to at least one of Claims 1 to 8, **characterized in that** from 2 to 10% by weight, preferably 4% by weight, of disintegrants are added to the outer phase on tableting of the mixture.

10. Process according to at least one of Claims 1 to 9, **characterized in that** from 0.3 to 2.5% by weight, preferably 1% by weight, of a flow regulator are added to the outer phase on tableting of the mixture.

## Revendications

1. Procédé de production de formulations pharmaceutiques à désagrégation rapide avec des préparations contenant un extrait, **caractérisé en ce que**, dans une première étape, la poudre d'extrait est compactée à sec en même temps qu'un lubrifiant en des quantités de 0,5 - 10 % en masse au moyen d'un dispositif approprié en une masse, puis concassée en granulés, triée et les produits compactés obtenus sont compressés dans une seconde étape en formulations pharmaceutiques avec les adjuvants habituels, comme des agents de charge, lubrifiants, de délitement, de régulation de l'écoulement, et d'autres additifs.

2. Procédé selon la revendication 1 **caractérisé en ce qu'**un compacteur est utilisé comme dispositif pour la compression, tandis qu'un granulateur-tamis est utilisé pour le concassage et la granulation.

3. Procédé selon les revendications 1 et 2 **caractérisé en ce que**, lors de la compression de la poudre d'extrait dans le compacteur, on opère avec une pression appliquée de 20 à 120 kN, de préférence de 35 à 65 kN et en particulier de 42 à 48 kN et une vitesse de rotation des cylindres de 5 à 15 tours/min.

4. Procédé selon au moins l'une des revendications 1 à 3 **caractérisé en ce que** le compacteur comporte pour la précompression une vis de précompression qui fonctionne à une vitesse de rotation de la vis de 20 à 60 tours/min, de préférence de 25 à 35 tours/min.

5. Procédé selon au moins l'une des revendications 1 à 4 **caractérisé en ce que** la quantité d'extrait par dose unitaire et de 100 à 1000 mg.

6. Procédé selon au moins l'une des revendications 1 à 5 **caractérisé en ce que** la quantité de lubrifiant dans le produit compacté est de préférence de 2 à 5 % en masse, en particulier de 3 % en masse.

7. Procédé selon au moins l'une des revendications 1 à 6 **caractérisé en ce que** du stéarate de magnésium est utilisé comme lubrifiant.

8. Procédé selon au moins l'une des revendications 1 à 6 **caractérisé en ce qu'**un ester d'acide gras et de glycérol, également un ester partiel, de préférence le tristéarate de glycérol, est utilisé comme lubrifiant.

9. Procédé selon au moins l'une des revendications 1 à 8 **caractérisé en ce que**, lors de la transformation du mélange en comprimés, 2 à 10 % en masse, de préférence 4 % en masse d'agents de délitement sont ajoutés à la phase externe.

10. Procédé selon au moins l'une des revendications 1 à 9 **caractérisé en ce que**, lors de la transformation du mélange en comprimés, 0,3 à 2,5 % en masse, de préférence 1 % en masse d'un agent de régulation de l'écoulement sont ajoutés à la phase externe.
